# EUROPÄISCHE PATENTSCHRIFT

(11) **EP 3 025 780 B1**
(45) Veröffentlichungstag und Bekanntmachung des Hinweises auf die Patenterteilung: **06.06.2018**
(21) Anmeldenummer: 14003971.0
(22) Anmeldetag: 26.11.2014
(51) Int. Cl.: B01L 3/00

(54) **Inkubationswanne**
Incubation tray
Plateau d'incubation

(43) Veröffentlichungstag der Anmeldung: 01.06.2016
(73) Patentinhaber: Euroimmun Medizinische Labordiagnostika AG, 23560 Lübeck (DE)
(72) Erfinder: Kemsies, Detlef, 23619 Zarpen (DE); Schröder, Dieter, 23617 Stockelsdorf (DE); Nevermann, Stefan, 23560 Lübeck (DE)

(56) Entgegenhaltungen:
- WO-A2-2012/122301
- DE-U1-202009 009 980
- DE-U1-202012 004 404
- US-A1- 2003 138 354

## Beschreibung

Die vorliegende Erfindung betrifft eine längliche Inkubationsrinne mit zwei einander gegenüber liegenden Längswänden, einer Vertiefung und einem Boden, bestückt mit einem länglichen Teststreifen mit einer Rückseite und einer Vorderseite, wobei der Teststreifen mit seiner Rückseite dem Boden der Inkubationsrinne zugewandt ist und auf seiner Vorderseite mit wenigstens einem analytischen Reagenz beschichtet ist, weiter aufweisend ein Befestigungsmittel, über das die Inkubationsrinne mit wenigstens einer weiteren Inkubationsrinne zu einem Verbund von Inkubationsrinnen verbunden werden kann, dadurch gekennzeichnet, dass auf einer der Längswände ein waagerecht nach außen stehender Auflagerahmen angebracht ist. Auf dem Gebiet der medizinischen Labordiagnostik sind unterschiedliche Testsysteme bekannt, mit denen Patientenproben auf das Vorhandensein spezifischer Antikörper untersucht werden können. Durch solche Nachweise ist es möglich, Rückschlüsse auf das Auftreten von Krankheiten zu ziehen, die zusammen mit derartigen spezifischen Antikörpern auftreten. Die Krankheit kann als Folge der Bildung von Autoantikörpern auftreten oder die Antikörper werden als Reaktion auf die Krankheit gebildet, beispielsweise als Reaktion auf das Eindringen von krankheitserregenden Viren. Beispielhafte Krankheiten umfassen Infektionen, entzündliche Krankheiten wie rheumatoide Krankheiten, Stoffwechselkrankheiten wie Diabetes und neurologische Erkrankungen.

üblicherweise erfolgt der Nachweis von diagnostisch relevanten Antikörpern im Wege der sogenannten Stufendiagnostik, bei der zunächst ein empfindliches Screening und dann eine spezifische Bestätigung durchgeführt werden. In der Routineserologie werden hierbei für das Screening oftmals ELISA (Enzyme-linked immunosorbent assay) verwendet, während als Bestätigungstest vornehmlich Immuno-Blotstreifen, insbesonderer Westernblot-Streifen, Dot Blot-Streifen oder Linienblot-Streifen eingesetzt werden.

Da der Bedarf an derartigen Testsystemen erheblich ist und die zum Einsatz kommenden Reagenzien oft hochpreisig, schwer zu beschaffen und nur in geringen Mengen verfügbar sind, ist eine Vereinfachung und Optimierung der Abläufe bei der Durchführung des jeweiligen Tests unerlässlich. Insbesondere müssen die verwendeten analytisch-diagnostischen Gerätschaften die möglichst störungsfreie und einfache parallele Durchführung zahlreicher Tests im Hochdurchsatzmaßstab ermöglichen. Jede Vereinfachung von Verfahrens-, Wartungs- oder Reinigungsschritten, jeder Ausschluß von noch so geringen Gefahrenquellen und jede Minimierung der Mengen der einzusetzenden Reagenzien führt für den Anwender zu erheblichen Einsparungen.

Besonderes Augenmerk ist dabei auf die Inkubationsrinnen zu richten, in denen die genannten Teststreifen bei der Durchführung der Tests mit verschiedenen Reagenzien unter verschiedenen Bedingungen inkubiert werden.

Vorrichtungen mit Inkubationsrinnen, die für derartige Testsysteme eingesetzt werden, können allerdings auch auf auf anderen technischen Gebieten eingesetzt werden, bei denen Teststreifen oder andere streifenförmige Träger in einer Flüssigkeit zu inkubieren sind. Beispielsweise werden Teststreifen auch in der chemischen Analytik zur einfachen Bestimmung der Konzentration des pH-Wertes oder von anorganischen Ionen verwandt. Auch bei der Fertigung von Teststreifen o.ä. Trägern kann es notwendig sein, diese einzeln in einer Flüssigkeit zu inkubieren.

Im Stand der Technik sind eine Reihe von Inkubationstinnen beschrieben, die für verschiedene analytische und diagnostische Tests eingesetzt werden können.

Die DE 20 2012 004 404 U1 offenbart eine Inkubationsrinne in einer Vorrichtung zur Untersuchung einer Patientenprobe, die mit einem länglichen Teststreifen bestückt ist, wobei der Teststreifen mit seiner Rückseite dem Rinnenboden zugewandt in die Inkubationsrinne eingelegt wird. Zur Handhabung wenigstens zweier Inkuabtionsrinnen ist ein Tablett mit einer Vielzahl von Aufnahmen vorgesehen, in die eine Inkubationsrinne und/oder ein Verbund wenigstens zweier Inkubationrinnen einlegbar ist. Zusätzlich zur Fixierung des Verbundes durch das Tablett mit den Aufnahmen kann die Inkubationsrinne an einer Längsseite ein Befestigungselement aufweisen, über das sie mit einer weiteren Inkubationsrinne verbunden werden kann, beispielsweise eine Schnapp- oder Clip-Verbindung.

Die DE 20 2012 004 404 U1 offenbart jedoch nicht, dass wenigstens zwei Inkubationsrinnen im verbundenen Zustand unabhängig von einem Tablett über eine Kombination aus Befestigungselementen an beiden Längsseiten der Inkubationswanne sowie einen Auflagerahmen miteinander verbunden sein können.

So kann ein Verbund der in der DE 20 2012 004 404 U1 beschriebenen Inkubationswannen nicht unabhängig von dem Tablett bewegt werden. Die Aufnahmen in dem Tablett sind zur Fixierung und Stabilisierung des Verbundes erforderlich. Ein Halteelement lediglich an einer Längsseite kann bestenfalls zwei kleine Inkubationswannen mit geringem Gewicht zusätzlich fixieren, nicht jedoch einen größeren Verbund, der unabhängig vom Tablett zu bewegen ist.

Zwischen den in der DE 20 2012 004 404 U1 offenbarten Inkubationrinhen liegen Spalten vor, durch die verpritzte Flüssigkeit durch den Verbund auf das Tablett und die Aufnahmen darin fließen kann. Dies bedingt einen hohen Aufwand bei der Reinigung oder die Notwendigkeit, das Tablett mit den Aufnahmen häufig auszutauschen.

Weiterhin offenbart die DE 20 2012 004 404 U1 eine Inkubationsrinne, die mit einem Teststreifen bestückbar ist. Das Aufschwimmen wird in einer Ausführungsform dadurch verhindert, dass der Teststreifen mit Hilfe von Haftkleber am Boden der Inkubationsrinne fixiert wird. Damit ist im Falle eines reversiblen Fixierens jedoch das Risiko verbunden, dass sich der Streifen bei längerer Inkubation in wässriger Lösung löst. Bei einem unlösbaren Haftkleber kann der Streifen der Vorrichtung nach der Analyse hingegen nicht entnommen und zur Dokumentation im getrockneten Zustand aufgehoben werden.

Als Alternativlösung offenbart die DE 20 2012 004 404 U1 die Möglichkeit, die Inkubationsrinne im Bodenbereich mit gegenüberliegenden Rastnasen zu versehen. Damit ist jedoch nur die Fixierung von relativ festen Teststreifen möglich. Die handelsüblichen weichen Teststreifen bieten nicht genug Widerstand, um das Einrasten von Rastnasen aus Kunststoffen zu ermöglichen. Es besteht weiterhin die Gefahr, dass die Streifen beim Einrasten beschädigt werden, wie in der DE 20 2012 004 404 U1 ausdrücklich erwähnt wird. Auch ist eine Entfernung aus der Inkubationsrinne nach dem Einrasten der Rastnasen umständlich, wenn nicht unmöglich.

Schließlich offenbart die DE 20 2012 004 404 U1 die Verwendung von Spangen zur Fixierung von Teststreifen in Bodennähe. Durch den Verschließmechanismus, in den die Streifen eingeklemmt werden können, besteht auch in dieser Variante die Gefahr von Beschädigungen. Schließlich verdeckt eine Spange den Teststreifen in der Aufsicht wenigstens teilweise. Durch das Verdecken und durch Schattenbildung wird die Bilderfassung und anschließende automatische Diagnose erschwert.

Die der Erfindung zu Grunde liegende Aufgabe besteht darin, ein System mit flexibel verbindbaren Inkubationsrinnen zu schaffen, in dem die Inkubationsrinnen im verbundenen Zustand unabhängig von einem Tablett als Einheit bewegt werden können, wobei die Gefahr des Auseinanderfallens des Verbunds gegenüber den im Stand der Technik beschriebenen Systemen reduziert ist.

Eine weitere der vorliegenden Erfindung zu Grunde liegende Aufgabe besteht darin, ein System mit flexibel verbindbaren Inkubationsrinnen zu schaffen, das gegenüber den im Stand der Technik beschriebenen Systemen einen verringerten Reinigungs- und Wartungsaufwand bedingt.

Der vorliegenden Erfindung daher weiterhin die Aufgabe zu Grunde, eine verbesserte Inkubationsrinne bereitzustellen, die eine Inkubation von Teststreifen ermöglicht, wobei die Teststreifen so am Boden fixiert sind, dass sie bei der Inkubation in wässriger Lösung von allen Seiten bedeckt sind, auf der anderen Seite nach der Inkubation aber leicht entnommen werden können.

Gleichzeitig soll die Inkubationsrinne das Anfertigen hochqualitativer Bildaufnahmen der Streifen nach der Inkubation ermöglichen, die anschließend manuell oder automatisch ausgelesen und zur Diagnose ausgewertet werden können.

Die Fixierung der Teststreifen soll dabei derart erreicht werden, dass eine Beschädigung der Teststreifen, die häufig mit hochpreisigen, aufwändig herzustellenden analytischen Reagenzien, nahezu ausgeschlossen ist.

Diese und weitere Aufgaben werden durch den Gegenstand der vorliegenden Anmeldung und insbesondere auch durch den Gegenstand des beigefügten unabhängigen Anspruchs gelöst, wobei sich Ausführungsformen aus den Unterarisprüchen ergeben.

In einem ersten Aspekt wird die Aufgabe gelöst durch eine längliche Inkubationsrinne mit zwei einander gegenüber liegenden Längswänden, einer Vertiefung und einem Boden, bestückt mit einem länglichen Teststreifen mit einer Rückseite und einer Vorderseite,
wobei der Teststreifen mit seiner Rückseite dem Boden der Inkubationsrinne zugewandt ist und auf seiner Vorderseite mit wenigstens einem analytischen Reagenz beschichtet ist,
weiter aufweisend ein Befestigungsmittel, über das die Inkubationsrinne mit wenigstens einer weiteren Inkubationsrinne zu einem Verbund von Inkubationsrinnen verbunden werden kann,
wobei auf einer der Längswände ein, bevorzugt waagerecht, nach außen stehender Auflagerahmen angebracht ist

In einer bevorzugten Ausführungsform verdeckt der Auflagerahmen die Vertiefung nicht.

In einer weiteren bevorzugten Ausführungsform sind die Längswand mit dem nach außen stehenden Auflagerahmen, der Auflagerahmen selbst und die andere Längswand derart aneinander angepasst, dass der Auflagerahmen der Inkubationsrinne im Verbund auf der anderen Längswand der weiteren Inkubationsrinne aufliegt.

In einer weiteren bevorzugten Ausführungsform erstreckt sich der Auflagerahmen entlang der Längsachse der Inkubationsrinne wenigstens so weit wie die Vertiefung.

In einer weiteren bevorzugten Ausführungsform befindet sich auf der anderen Längswand eine Rahmenaussparung, bevorzugt in der Form des im Verbund darauf aufliegenden Auflagerahmens.

In einer weiteren bevorzugten Ausführungsform ist das Ende des Auflagerahmens entlang der Querachse so bemessen, dass er an die Längswand der weiteren Inkubationswanne anschließt und deren vertikale Verlängerung bildet.

In einer weiteren bevorzugten Ausführungsform umfasst das Befestigungsmittel wenigstens eine Einheit mit einem vorstehenden Element auf der einen Längsseite der Inkubationsrinne und einem aufnehmenden Element auf der anderen Längsseite der Inkubationsrinne.

In einer weiteren bevorzugten Ausführungsform befindet sich das vorstehende Element des Befestigungsmittels auf der gleichen Längsseite der Inkubationsrinne wie der Auflagerahmen.

In einer weiteren bevorzugten Ausführungsform gestattet das Befestigungsmittel eine Verbindung zwischen der Inkubetionsrinne und der weiteren Inkubationsrinne, die mehrfach eingerichtet und wieder gelöst werden kann.

In einer weiteren bevorzugten Ausführungsform ist an jedem Ende der Inkubationsrinne ein Befestigungsmittel, bevorzugt ein vorstehendes Element auf der einen Längsseite und ein an letzteres angepasstes aufnehmendes Element auf der anderen Längsseite, angeordnet.

In einer weiteren bevorzugten Ausführungsform erstreckt sich die Vertiefung nicht über die gesamte Länge der Inkubationsrinne, sondern es befindet sich an beiden Längsenden der Inkubationsrinne ein Fortsatz, der jeweils eine Einheit des Befestigungsmittel beherbergt.

In einer weiteren bevorzugten Ausführungsform weist jeder Fortsatz einen Kopf auf, der über ein Verbindungsstück mit der Vertiefung verbunden ist,
wobei das Verbindungsstück vertikal in der Ebene des Auflagerahmens angeordnet und sich unter ihm eine zur Längsachse der Inkubationsrinne senkrecht angeordnete Aushöhlung entsteht.

In einer weiteren bevorzugten Ausführungsform umfasst das Befestigungsmittel ein vorstehendes Element auf der einen Längsseite und ein an letzteres angepasstes aufnehmendes Element auf der anderen Längsseite, wobei
das vorstehende Element und die Längswand mit dem Auflagerahmen an der einen Längsseite der Inkubationrinne angeordnet sind und
das aufnehmende Element und die andere Längswand an der anderen Längsseite der Inkubationsrinne angeordnet sind
und sie bevorzugt derart aneinander angepasst sind, dass die Position von wenigstens zwei gleich beschaffenen Inkubationsrinnen im Verbund sowohl durch das Einrasten des vorstehenden Elements in das aufnehmende Element als auch durch das Auflegen des Auflagerahmens auf die Rahmenaussparung der anderen Längswand fixiert ist.

In einer weiteren bevorzugten Ausführungsform umfasst das Befestigungsmittel ein vorstehendes Element auf der einen Längsseite und ein an letzteres angepasstes aufnehmendes Element auf der anderen Längsseite, wobei
das aufnehmende Element des Befestigungsmittels nach oben und in Richtung der Außenseite der Längswanne offen und
so gestaltet ist, dass das vorstehende Element von oben in das aufnehmende Element eingeführt wird, bis ersteres auf den Boden des letzteren aufsetzt.

In einer weiteren bevorzugten Ausführungsform umfasst das Befestigungsmittel ein vorstehendes Element auf der einen Längsseite und ein an letzteres angepasstes aufnehmendes Element auf der anderen Längsseite,
und wobei das vorstehende Element und das aufnehmende Element derart aneinander angepasst sind, dass beim Verbinden das erstere mit einem hörbaren Geräusch in das letztere einrastet. Die Inkubationsrinne umfasst weiter den Teststreifen, der bevorzugt einen aus der Vertiefung ragenden Fortsatz zum Herausziehen aufweist.

Erfindungsgemäß kann die Inkubationsrinne ein Befestigungsmittel aufweisen, das geeignet ist, um die Inkubationsrinne mit wengistens einer weiteren Inkubationsrinne zu einem Verbund von Inkubationsrinnen zusammenzufügen. Im Verbund sind die Inkubationsrinnen als Einheit handhabbar. Sie können beispielsweise zusammen in ein Gerät eingesetzt, ausgegossen oder gewaschen werden, ohne dass dazu jede der Inkubationsrinnen im Verbund einzeln bewegt werden muss. Der Verbund umfasst wenigstens 2, bevorzugt wenigstens 3, 4, 5, 10, 12, 15, 20, 24, 36 oder 50 Inkubationsrinnen. Die Zahl der Inkubationsrinnen im Verbund ist Im Wesentlichen durch die Aufnahmekapazität der Geräte begrenzt, in denen der Verbund und in den Einzelrinnen befindliche Proben prozessiert werden. Ebenso wird die Zahl der Inkubationsrinnen so begrenzt, dass der Verbund ausreichend stabil und nicht zu schwer wird. Bevorzugt besteht der Verbund aus im Wesentlichen oder tatsächlich identischen Inkubationsrinnen. Dabei kommt es jedoch lediglich auf die Berührungsflächen der Inkubationsrinnen an, die so gestaltet sein müssen, das das Befestigungsmittel und sonstige relevante Elemente wie der erfindungsgemäße Auflagerahmen - der einen Wand das Zusammenfügen mit der im Verbund jeweils nächsten Inkubationsrinne gestattet. Sonstige Merkmale der einzelnen Inkubationsrinnen, beispielsweise ihr Inhalt, ihre Kennzeichnung, ihre Breite o.ä, können variiert werden. Für analytische oder diagnostische Anwendungen kann jede der Inkubationsrinnen, auch wenn sie ansonsten gleich beschaffen oder gar identisch sind, eine Kennzeichnung aufweisen, die die darin zu untersuchende Probe einem Experiment bzw. Patienten zuordnet.

Das bevorzugte Befestigungsmittel weist ein vorstehendes Element und ein aufnehmendes Element (im Stand der Technik häufig als "männliche" bzw. "weibliches" Teil bezeichnet) auf, die ineinander gesteckt werden und einrasten, so dass die Verbindung zwischen den Inkubationsrinnen ausgebildet wird. Das vorstehende Element weist am oder nahe seinem Ende, das von der Inkubationsrinne wegzeigt, wenigstens eine Stelle auf, die breiter ist als das der Inkubationsrinnen zugewandte Ende. Das aufnehmende Element ist an diese Form angepasst, insbesondere dadurch, dass es nach oben zur Aufnahme des vorstehenden Elements und in Richtung der Außenseite der Längsrinne offen ist, so dass die beiden Elemente nach dem Einrasten nicht durch eine Bewegung in ausschließlich horizontaler Richtung voneinander getrennt werden können, sondern ein Herausheben oder -drehen mit einer vertikalen Komponente erforderlich ist, das vorstehende Element also von oben in das aufnehmende Element eingeführt wird, wobei die endgültige vertikale Position des vorstehenden Elements bevorzugt durch den Boden des aufnehmenden Elements bestimmt wird, auf den das vorstehende Element beim Einrasten aufsetzt. Beispielhafte Befestigungsmittel sind in verschiedenen Variationen im Stand der Technik offenbart, beispielsweise die als Schwalbenschwanz bezeichnete Verbindung.

Eine zum Verbund führende Verbindung zwischen Inkubationsnrinnen mit Befestigungsmitteln wie der klassischen Schwalbenschwanzverbindung kann man mehrfach einrichten und danach wieder lösen. In einer alternativen Ausführungsform weist die Inkubationsrinne ein Befestigungsmittel auf, mit dem die Inkubationswanne nur einmal mit einer weiteren Inkubationswanne zusammengefügt werden kann. Dies lässt sich zum Beispiel dadurch verwirklichen, dass im vorstehenden Element eine Sollbruchstelle eingefügt ist, während letzteres sehr fest und mit normalem Kraftaufwand nicht lösbar in das aufnehmende Element einrastet. Das Lösen der Verbindung ist dann nur unter Zerstörung des Befestigungsmittels möglich.

Eine besonders bevorzugte Variante des Befestigungsmittels ist die Schwalbenschwanzverbindung mit begradigter Kante. Dabei verläuft, von oben betrachtet, die dem Längsende der Inkubationsrinne zugewandte Kante des vorstehenden Elements senkrecht zur Längsachse der länglichen Inkubationsrinne. Der Vorteil besteht darin, dass die Wannen mit einer vertikaldrehenden Bewegung mit einander unter Ausbildung des Verbunds zusammengefügt bzw. wieder voneinander gelöst werden. Dies ermöglichst dem Anwender gegenüber der klassischen Schwalbenschwanzverbindung ein komfortableres Lösen von Inkubationsrinnen aus dem Verbund.

Bevorzugt weisen in dem erfindungsgemäßen Verbund sämtliche Inkubationsrinnen das gleiche Befestigungsmittel mit komplementären Elementen auf. Es ist jedoch auch möglich, dass verschiedene Inkubationsrinnentypen mit miteinander nicht oder nur in bestimmten Kombinationen kompatiblen Befestigungsmitteln im Verbund enthalten sind, beispielsweise um bestimmte Anordnungen oder Muster von verschiedenen Typen von Inkubationsrinnen innerhalb eines größeren Verbundes festzulegen, beispielsweise das Alternieren zweier Typen von Inkubationsrinnen. Beispielsweise kann Typ A einer Inkubationsrinne an der einen Seite das aufnehmende Element eines ersten Befestigungsmittels, z.B. einer Schwalbenschwanzverbindung, aufweisen und an der anderen Seite das vorstehende Element eines zweiten Befestigungsmittels, das z.B. einem Kugelgelenk nachgeahmt Ist. Die Inkubationsrinne vom Typ A kann damit nicht direkt mit einer weiteren Inkubationsrinne vom Typ A zusammengefügt werden.

In einer bevorzugten Ausführungsform wird unter dem Begriff "Teststreifen", wie hierin verwendet, ein bevorzugt länglicher Träger verstanden, der gegen übliche Lösungsmittel, insbesondere auf wässriger Basis, chemisch ausreichend inert ist und mit einem Reagenz beschichtet ist, das für eine erwünschte chemische Reaktion, insbesondere ein analytisches Verfahren, besonders bevorzugt ein labordiagnostisches Verfahren, geeignet ist. Beispielsweise kann es sich um einen Nitrocellulosestreifen mit einem Antigen handeln. In einer bevorzugten Ausführungsform, wird unter dem Begriff "länglich", wie hierin verwendet, dass das Längenverhältnis der längeren zur kürzeren Seite in der Reihenfolge zunehmender Bevorzugung wenigstens 1,5:1, 2:1, 3:1, 4:1, 5:1, 7,5:1, 10:1, 15:1 oder 20:1 beträgt. In einer weiteren bevorzugten Ausführungsform wird unter dem Begriff "beschichtet", wie hierin verwendet, verstanden, dass das Reagenz derart mit dem Teststreifen verbunden ist, dass eine mit der Vorderseite des Streifens in Kontakt stehende Flüssigkeit auch mit Kontakt mit dem Reagenz tritt. Beispielsweise kann das Reagenz auf der Oberfläche der. Vorderseite aufgeblottet sein, oder der Teststreifen kann an jeder Stelle eine im Wesentlichen einheitliche Konzentration des Reagenz aufweisen. Geeignete Teststreifen sind im Stand der Technik beschrieben und im Handel erhältlich, beispielsweise Linienblots von der Firma EUROIMMUN, Lübeck. In einer bevorzugten Ausführungsform wird unter dem Begriff "analytische Reagenz", wie hierin verwendet, eine chemische Verbindung verstanden, die mit einem in einer Probe oder sonstigen zu untersuchenden wässrigen Lösung enthaltenen Analyten chemisch oder physikalisch reagiert. Diese Reaktion kann nachgewiesen werden. Beispielsweise handelt es sich bei dem analytischen Reagenz um ein Antigen, besonders ein epitophaltiges Polypeptid, an das ein nachzuweisender Antikörper, bevorzugt Autoantikörper, aus einer zu untersuchenden Probe bindet, worauf der entstehende Antigen-Antikörper-Komplex mittels eines weiteren, enzymkonjugierten Antkörpers nachgewiesen werden kann. Alternativ kann es sich bei dem analytischen Reagenz z.B. um einen pH-abhängigen Farbstoff handeln, der eine bestimmte indikative Farbe annimmt, wenn er gegenüber einer Lösung mit einem bestimmten pH-Wert exponiert wird.

Ir einer bevorzugten Ausführungsform wird unter dem Begriff "Inkubationsrinne", wie hierin verwendet, ein bevorzugt flüssigkeitsdichter Behälter mit einem Boden, zwei Längswänden und zwei Querwänden verstanden. Die Inkubationswanne ist länglich ausgestaltet, um einen Teststreifen aufnehmen zu können. Bei einer Inkubationswanne von rechteckigem Umfang beträgt das Längenverhältnis von Querwand zu Längswand wenigstens 1:2, bevorzugter 1:3, 1:4, 1:5, 1:6, 1:7, 1:8, 1:9 oder 1:10. Bevorzugt besteht die Inkubationswanne aus einem gegenüber wässrigen Lösungen resistenten Material, beispielsweise Polystyrol, Polyethylen oder Polypropylen. In einer bevorzugten Ausführungsform ist die Inkubationswanne mit einem Teststreifen bestückt und, optional, in einer Verpackung enthalten. In einer weiteren bevorzugten Ausführungsform enthält die Inkubationsrinne neben dem Teststreifen eine Flüssigkeit. Dabei kann es sich um eine zu untersuchende Probe, eine Waschlösung oder eine Lösung mit chemischen Reagenzien, Konservierungsmitteln oder Analyten handeln.

Die Inkubationsrinne kann rein manuell durch händisches Einbringen und Entfernen der benötigten Reagenzien für chemische Reaktionen, besonders analytische oder diagnostische Tests verwendet werden. Bevorzugt wird die Inkubationsrinne bzw. der Verbund aber in eine Vorrichtung eingebracht, die zahlreiche, idealerweise alle Schritte vollautomatisch ausführt, möglichst ohne dass die Anwesenheit von fachkundigem Personal erforderlich ist. Eine solche Vorrichtung ist zur Bevorratung sowie zum Einbringen und Absaugen von geeigneten Puffern und Reagenzien ausgestattet, idealerweise zusätzlich zur Anfertigung geeigneter Bildaufnahmen vom voll prozessierten Teststreifen.

Die Inkubationsrinne ist bevorzugt konisch ausgestaltet, um die zur Bedeckung des Teststreifens notwendige Mindestvolumina an Flüssigkeit zu minimieren.

Bevorzugt weist die Inkubationsrinne einen Fortsatz an wenigstens einem, bevorzugt an den beiden Längsenden auf. Das hat den Vorteil, dass ein Schutz gegen das Verspritzen von Flüssigkeit in Richtung der Längsenden der Inkubationsrinne gegeben ist. Der Fortsatz, besonders sein Kopf, Ist ein bevorzugter Ansatzpunkt für die Positionierung von Elementen das Befestigungsmittels. Eine Aushöhlung unter dem Verbindungsstück kann für die Auflage der Inkubationsrinne in eine Apparatur oder Transport- bzw. Inkubationswanne dienen, die eine entsprechend passende Erhöhung aufweist. Die Inkubationsrinnen bzw. der Verbund ist dann innerhalb der Apparatur oder Wanne fixiert und kann nicht verrutschen, wenn die Inkubationsrinne bzw. der Verbund geschwenkt oder geschüttelt wird.

Optional kann die Inkubationsrinne bzw. der Verbund mit einer Abdeckung versehen werden. Dabei kann es sich um eine durchgehende Folie handeln, die den gesamten Verbund abdeckt, oder um einzelne, auf die Form der Inkubationsrinne abgestimmte Deckel.

Erfindungsgemäß kann die Inkubationsrinne weiterhin einen auf einer der Längswände angebrachten, bevorzugt waagerecht nach außen stehenden Auflagerahmen aufweisen. Dieser Auflagerahmen, der bevorzugt nicht die Vertiefung überdeckt, um die optische Betrachtung bzw. Bildaufnahme des Teststreifens nicht zu erschweren, kann eine beliebige Form annehmen, solange er auf die Längswand der weiteren Inkubationswand im Verbund so zugeschnitten ist, dass er im Verbund auf dieser aufliegt und damit eine in quer zur Längsachse der Inkubationsrinne lückenlose Abdeckung derart schafft, dass eine auf den Verbund auftropfende Flüssigkeit entweder auf den Rahmen oder die Vertiefung trifft. Bevorzugt weist die Längswand, auf der der Auflagerahmen aufliegt, eine an ihn formangepasste Rahmenaussparung auf. Nicht als Teil des, aber analog zum Befestigungsmittel kann der Auflagerahmen dabei als vorstehendes Element und die Rahmenaussparung als aufnehmendes Element fungieren. Der Auflagerahmen steht bevorzugt in einem Winkel von 60 bis 120 °, bevorzugter 75 bis 105 °, noch bevorzugter 80 bis 100 °, am bevorzugtesten 90 °, d.h. waagerecht, von der senkrecht vom Boden abstehenden Längswand ab. Bei einer konischen Längswand wird der Winkel entsprechend einer an gleicher vertikaler Position mit dem Außenrahmen verbundenen senkrechten Längswand bemessen. Entscheidend ist, dass der Außenrahmen so gestaltet ist, dass er im Verbund auf der Längswand der weiteren Inkubationswand aufliegt.

Bevorzugt erstreckt sich der Auflagerahmen entlang der Längsachse der Inkubationsrinne wenigstens so weit wie die Vertiefung. Damit wird gewährleistet, dass seitlich verspritzte Flüssigkeit nicht zwischen den Inkubationsrinnen eines Verbundes durchläuft. Der Auflagerahmen ist bevorzugt parallel zum Boden der Inkubationsrinnen angeordnet. In einer bevorzugten Ausführungsform ist seine Unterseite derart ausgestaltet, dass er parallel auf der anderen Längswand der nächsten Inkubationrinne im Verbund aufliegt, seine Oberseite ist jedoch nach Art eines Pultdachs geneigt, so dass verspritzte Flüssigkeit in die Vertiefung zurückläuft.

Der Auflagerahmen bildet bevorzugt eine vertikale Verlängerung der Längswand, auf der er aufliegt. Das bedeutet, dass er eine Verlängerung der Wand in dem Winkel bildet, indem die Wand sich vom Boden der Vertiefung erhebt, beispielsweise 90 ° bei einer senkrecht stehenden Wand und einem Winkel von mehr als 90 °C bei einer konisch ausgestalteten Inkubationsrinne, entsprechend dem Winkel, in dem die Längswand vom Boden der Vertiefung absteht.

Weiterhin kann die erfindungsgemäße Inkubationswanne ein System umfassend wenigstens zwei Halteelemente umfassen, die geeignet sind, den Teststreifen für die Durchführung von chemischen und biologischen Reaktionen, insbesondere labordiagnostischen Verfahren, in geeigneter Weise in der Inkubationsrinne zu positionieren. Dazu gehört insbesondere, dass ein Aufschwimmen, welche zur Austrocknung und Abkehr wenigstens einer Seite des Teststreifens von reagenzhaltigen Flüssigkeiten führt, sowie das Umdrehen des Teststreifens, welches die Betrachtung oder Bildaufnahme stört, verhindert werden muss.

Erfindungsgemäß wird die Aufgabe durch Halteelemente in der Inkubationsrinne gelöst, die aus wenigstens einer Längswand hervortreten, wobei bevorzugt ist, dass die beiden Längswände der Inkubationsrinne jeweils wenigstens ein Halteelement aufweisen. Jedes Halteelement besteht aus einem in Bodennähe aus der Längswand hervorstehenden Vorsprung. Dieser ragt so weit ins Innere der Vertiefung, dass er, insbesondere in Kombination mit einem weiteren Halteelement, bevorzugt an der gegenüberliegenden Längswand, geeignet ist, die Bewegung eines darunter eingeführten Teststreifens in vertikaler Richtung insoweit zu begrenzen. Bei der Durchführung des Tests kann dann so viel Flüssigkeit in die Inkubationsrinne gegeben werden, dass der Streifen in ausreichendem Maße bedeckt ist. Die Menge der Flüssigkeit kann durch geeignete Anordnungen minimiert werden, die ein Schwenken der Inkubationsrinne gestatten, wobei die Flüssigkeit von einem zum anderen Längsende zum anderen und wieder zurück fließt. Derartige Anordnungen sind im Stand der Technik beschrieben, beispielsweise in der EP 08 169 465.5 oder EP 2191893.

Unterhalb des Vorsprungs weist das Halteelement eine Aussparung auf, die so groß ist, dass der Teststreifen darunter zwar Platz findet und bevorzugt bei einer Bewegung der Inkubationsrinne zur optimalen Exponierung gegenüber der umgebenden Flüssigkeit und darin enthaltener Reagenzien leichte Aufwärts- und Abwärtsbewegungen ausführen, sich aber nicht um seine Längsachse drehen kann. Je nach Breite des Streifens und der Inkubationsrinne kann die Aussparung entlang der Querachse der Inkubationsrinne vor der Längswand enden. Im Gegensatz dazu ist es auch möglich, dass die Aussparung entlang der Querachse der Inkubationsrinne bis zur Längswand reicht, d.h. sie umfasst die gesamte Strecke zwischen der Längswand, aus der der Vorsprung herausragt, und dem Ende des Vorsprungs im Inneren der Inkubationsrinne. Der Teststreifen kann sich für den Fall, dass er auf beiden Seiten durch derartig beschaffene Halteelemente positioniert wird, über die gesamte Breite der Inkubationsrinne bewegen, ist jedoch durch die Vorsprünge an einem Aufschwimmen bzw. Drehen gehindert.

Mit Hinblick auf die Länge des Vorsprungs eines Halteelements ist es essentiell, dass dieser keinesfalls bis zur gegenüberliegenden Längsseite reicht. Bevorzugt entspricht die Länge des Vorsprungs über der Querachse der Inkubationsrinne am Boden weniger als 75, noch bevorzugter weniger als 50, 45, 40, 30, 25 oder 20 % der Breite der Inkubationsrinne am Boden. In einer weiteren bevorzugten Ausführungsform ist ein in der Mitte der Inkubationsrinne liegender Teststreifen, dessen Breite 25, bevorzugter 50, noch bevorzugter 70 % der Breite der Inkubationswanne aufweist, über höchstens 30 % seiner Breite durch den Vorsprung des Halteelements überdeckt.

Die erfindungsgemäßen Halteelemente positionieren den Teststreifen besonders effektiv, wenn sie in Paaren angeordnet sind. Das ist bei zwei Halteelementen dann der Fall, wenn sie entlang der Längsachse der Inkubationsrinne auf gegenüberliegenden Längsachsen relativ zu den anderen Halteelementen so angeordnet sind, dass ihre Zusammengehörigkeit aufgrund ihrer räumlichen Nähe erkennbar ist. Dabei können die zwei Halteelemente eines Paares an den beiden Längsseiten einander gegenüber entlang der Längsachse der Inkubationsrinne diagonal versetzt oder auf der gleichen Höhe der Längsachse der Inkubationsrine entlang angeordnet sein. Bevorzugt beträgt der Abstand der beiden Halteelemente eines Paares entlang der Längsachse der Inkubationsrinne höchsten 40, bevorzugter, 30, bevorzugter, 20, bevorzugter 20, bevorzugter 10, bevorzugter 7,5, bevorzugter 5, bevorzugter 2,5 % der Länge der Vertiefung der Inkubationsrinne. Die Halteelemente können auch alternierend entlang der Längsachse auf beiden Seiten der Inkubationswanne angeordnet sein.

Das Halteelement kann die Form eines horizontal gespiegelten Buchstabens L annehmen. Besonders bevorzugt ist das Halteelement vertikal abgeschrägt, abgerundet oder in Form eines Schafts ausgeprägt. Auf diese Weise ist die schattenbildende Fläche verringert. Die verringerte Schattenbildung auf dem Teststreifen sorgt für eine bessere optische Zugänglichkeit, die eine verbesserte Erkennbarkeit bzw. Bildqualität von gewonnnenen Aufnahmen bedingt. Die Ausbildung mit einer vertikal abgeschrägten oder abgerundeten Form stellt einen besonders vorteilhaften Kompromiss zwischen Stabilität des Halteelementes und Minimierung der Schattenbildung dar. Die Halteelemente sind geeignet, um den Teststreifen in Bodennähe zu fixieren, worunter in einer bevorzugten Ausführungsform eine Position einem vertikalen Bereich verstanden wird, der die untersten 80, bevorzugter 60, bevorzugter 50, bevorzugter 40, bevorzugter 30, bevorzugter 20, bevorzugter 15, bevorzugter 10, bevorzugter 5 % der Höhe der Vertiefung der Inkubationsrinne umfasst.

Bevorzugt sind Inkubationsrinne und die Halteelemente in einem Stück gefertigt. Die Halteelemente stehen dann direkt aus den Längswänden der Inkubationsrinne hervor. Alternativ können die Halteelemente in Form eines Halteelementrahmens separat von der Inkubationsrinne bereitgestellt werden. Der Halteelementrahmen kann durch einen oder mehr als einen, bevorzugt mindestens zwei Halteelementrahmenfüße auf dem Boden der Inkubationsrinne aufgesetzt werden, wobei die Höhe der Halteelementrahmenfüße den Abstand des Halteelementrahmens vom Boden der Inkubationswanne bestimmt. Alternativ kann auch die Form bzw. der Umfang des Halteelementrahmens so angepasst werden, dass er bei einer konischen Inkubationsrinne direkt auf den Längswänden aufsetzt. Dazu ist der Halteelementrahmen enger als die Vertiefung nahe dem oberen Ende der Längswände, aber breiter als am Boden der Inkubationsrinne zu gestalten. Bevorzugt ist der Halteelementrahmen so beschaffen, dass er auf einem Niveau von weniger als 80, bevorzugter 60, bevorzugter 50, bevorzugter 40, bevorzugter 30, bevorzugter 20 oder bevorzugter 15 % der Höhe der Vertiefung der Inkubationsrinne positioniert ist. Sofern das Eigengewicht des Halteelementrahmens nicht genügt, kann er durch zusätzliche Gewichte beschwert werden.

Ein erfindungsgemäßes Verfahren kann einen oder mehr als einen der folgenden Schritte umfassen, bevorzugt, aber nicht notwendigerweise in der genannten Reihenfolge:
a) Bereitstellen der erfindungsgemäßen Inkubationsrinne umfassend den Teststreifen,
b) Einbringen von Probenmaterial in einer Flüssigkeit, bevorzugt wässrigen Lösung, in die Blotwanne, so dass dieses in Kontakt mit dem Teststreifen tritt,
c) Inkubation des Teststreifens in Anwesenheit des Probenmaterials,
d) Entfernung des Probenmaterials,
e) Waschen des Teststreifens
f) Einbringen von wenigstens einem chemischen Reagenz und Inkubation mit dem Teststreifen aus Schritt d),
g) Optional wenigstens einmalige Wiederholung von Schritt f) mit dem gleichen oder einem anderen chemischen Reagenz
h) Optional Waschen des Teststreifens
i) Optional Entnahme des Teststreifens
j) Optional Analyse des Teststreifens
k) Optional Trocknen und ggf. Archivieren des Teststreifens.

Im Folgenden wird die Erfindung unter Bezugnahme auf die Figuren anhand von Ausführungsbeispielen erläutert. Die beschriebenen Ausführungsformen sind in jeder Hinsicht lediglich beispielhaft und nicht als einschränkend zu verstehen, und verschiedene Kombinationen der angeführten Merkmale sind vom Umfang der Erfindung umfasst.

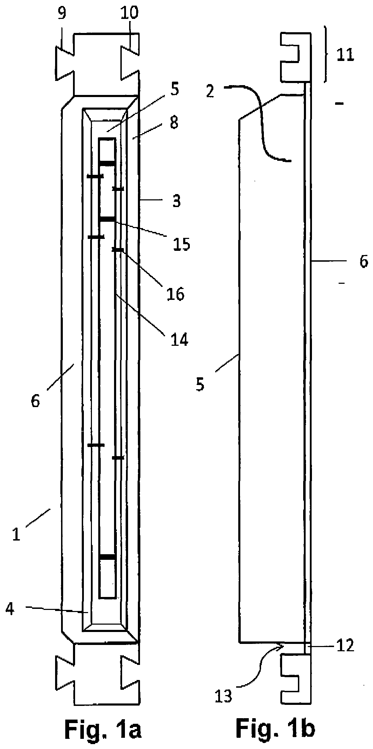

**Fig. 1a** zeigt eine von oben betrachtete erfindungsgemäße Inkubationsrinne (1) mit Längswänden ((3) und (2), letztere nicht sichtbar unter dem Auflagerahmen (6)) und einer Vertiefung (4), in der sich auf dem Boden (5) ein Teststreifen (14) mit Reagenz (15) befindet, der durch Halteelemente (16) fixiert ist. Die eine Längsseite weist einen Auflagerahmen (6) auf, die andere einen an einen passenden Auflagerahmen Rahmenaussparung (8). Dargestellt ist außerdem das vorstehende (9) und aufnehmende (10) Element des Befestigungsmittels.

Wie in **Fig. 1b** von der Seite gezeigt, befindet sich an den Längsenden jeweils ein Kopf (11) mit einem vorstehenden (9) und einem aufnehmenden Element (10). Der Kopf ist über ein Verbindungsstück (12), das darunter eine Aushöhlung (13) aufweist, mit den übrigen Teilen der Inkubationswanne verbunden.

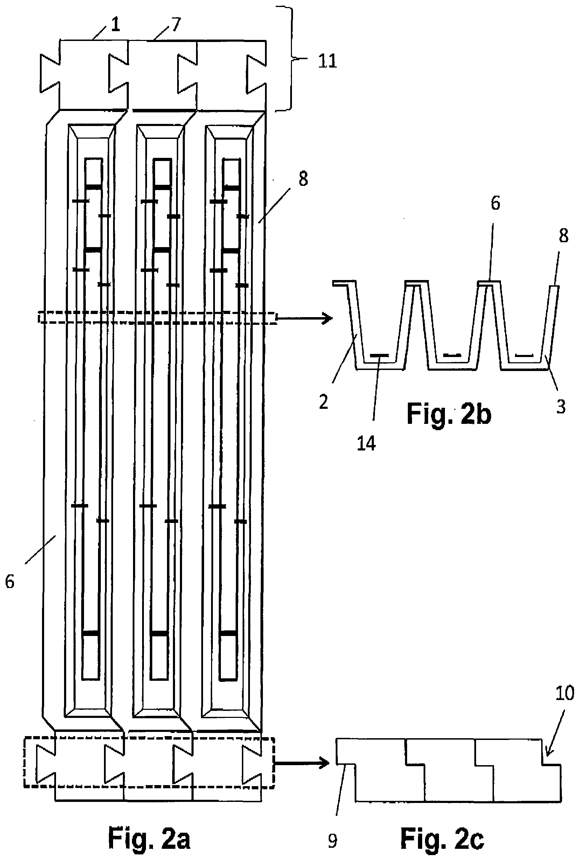

**Fig. 2a** zeigt einen erfindungsgemäßen Verbund von drei identischen Inkubationswannen, wie sie in **Fig. 1a** gezeigt sind. Der Verbund wird fixiert durch den in die Rahmenaussparung (8) passenden Auflagerahmen (6) sowie durch das in das aufnehmende Element (10) gesteckte und eingerastete vorstehende Element (9).

**Fig. 2b** illustriert im Querschnitt, wie der Auflagerahmen (6) so an die Rahmenaussparung (8) der nächstliegenden Inkubationswanne im Verbund angepasst ist, dass der Zwischenraum gegen ein mögliches Verspritzen von Flüssigkeit abgeschlossen und abgedichtet ist, besonders bei konisch geformten Inkubationsrinnen.

**Fig. 2c** illustriert im Querschnitt, wie vorstehendes Element (9) und aufnehmendes Element (10) im Verbund eingerastet sind.

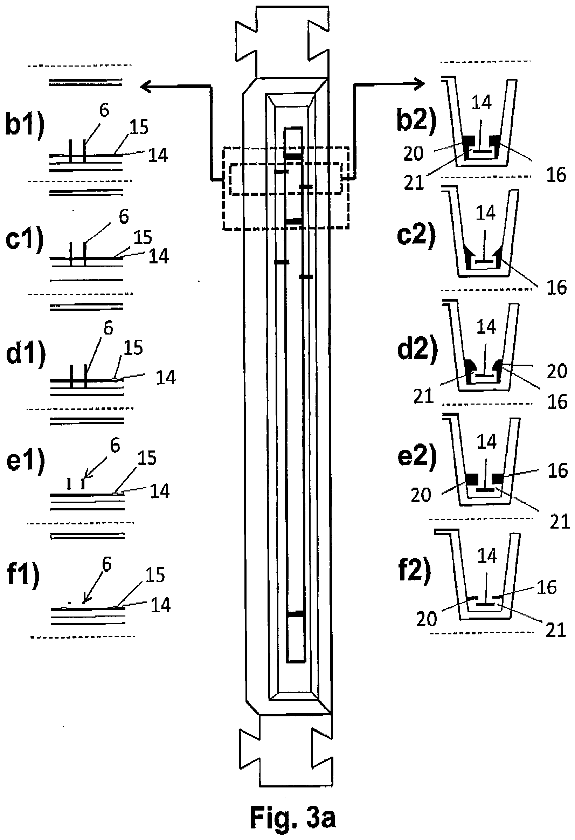

**Fig. 3** zeigt verschiedene erfindungsgemäße Ausgestaltungen des Halteelemente (6) ausgehend von der **Fig. 1a** entsprechenden Inkubationsrinne in **Fig. 3a****.** Kennzeichnend für alle erfindungsgemäßen Halteelemente ist der aus der oder aus Richtung der Längswand herausragende Vorsprung (20), unter dem sich eine Aussparung (21) befindet. Der Vorsprung begrenzt in vertikaler Richtung die Lage eines ein unter ihm eingeführten Teststreifens (14) mit einem Reagenz (15) durch ein Paar ausbildende Halteelemente auf gegenüberliegenden Längsseiten der Inkubationsrinne, Der Teststreifen wird auf diese Weise in einer Maximalhöhe am Boden gehalten wird, die der unteren Kante des Vorsprungs entspricht. Seine Lage in horizontaler Richtung beschränkt sich durch die Form der Aussparung (21) der Halteelemente.

Die **Fig. 3b1), 3c1), 3d1), 3e1)** und **3f1)** bzw. **3b2), 3c2), 3d2), 3e2)** und **3f2)** zeigen im Querschnitt entlang der Längsachse bzw. Querachse der Inkubationsrinne verschiedene Abwandlungen des Halteelementes, insbesondere mit vertikal abgeschrägtem Vorsprung **(3c1)** und **3c2)),** vertikal abgerundetem Vorsprung **(3d1)** und **3d2)),** Halteelemente, bei denen die Aussparung entlang der Querachse der Inkubationsrinne vor der Längswand endet **(3b2), 3c2)** und **3d2))** und, im Gegensatz zu den letzteren, Halteelemente, bei den denen die Aussparung bis zur Längswand reicht **(3e2)** und **3f2)).** Im letzteren Fall ist die Lage des Teststreifens in horizontaler Richtung ausschließlich durch die beiden Längswände begrenzt. Die **Figs. 3f1)** und **3f2)** zeigen in Form von Schäften ausgebildete Halteelemente.

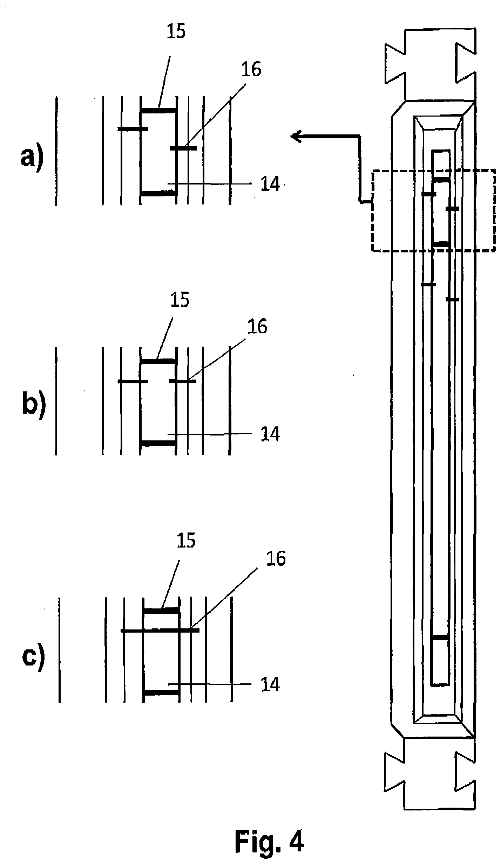

**Fig. 4** zeigt verschiedene Möglichkeiten zur Anordnung von zwei Halteelementen an gegenüberliegenden Längsseiten in Paaren anhand einer von oben betrachteten Inkubationsrinne, wobei die **Figs. 4a), 4b)** und **4c****)** die In **Figs. 1a** und **3a** dargestellte Variante in Vergrößerung **(****Fig. 4a****))** mit zwei möglichen Abwandlungen **(4b)** und **4c))** illustrieren. In Fig. **4a****)** sind die zwei Halteelemente des Paares an den beiden Längsseiten auf der gleichen Höhe der Längsachse der Inkubationsrine entlang angeordnet. In **Fig. 4b****)** sind die zwei Halteelemente des Paares an den beiden Längsseiten einander gegenüber entlang der Längsachse der Inkubationsrinne diagonal versetzt angeordnet. Leglich illustrativ zum Vergleich zeigt **Fig. 4c****)** eine nicht erfindungsgemäße Abwandlung, in der die zwei Halteelemente an den beiden Längsseiten auf der gleichen Höhe der Längsachse der Inkubationsrine entlang angeordnet und in Form einer Spange verbunden sind, so dass sie den Teststreifen überdecken.

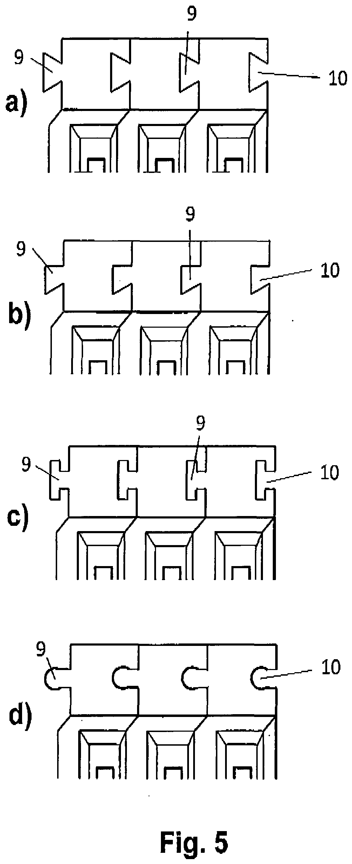

**Fig. 5** zeigt verschiedene Möglichkeiten zur Ausgestaltung des Befestigungsmittels mit einem vorstehenden Element (9) und einem aufnehmenden Element (10). **Fig. 5a****)** zeigt die sogenannte Schwalbenschwanzverbindung. **Fig. 5b****)** zeigt die Schwalbenschwanzverbindung mit einer begradigten Kante. **Fig. 5c****)** zeigt ein Befestigungsmittel mit stempelförmigem vorstehenden Element. **Fig. 5d****)** zeigt ein Befestigungsmittel, bei dem das vorstehende Element kreisförmig bzw. dreidimensional in Form eines Kugelgelenks ausgestaltet ist.

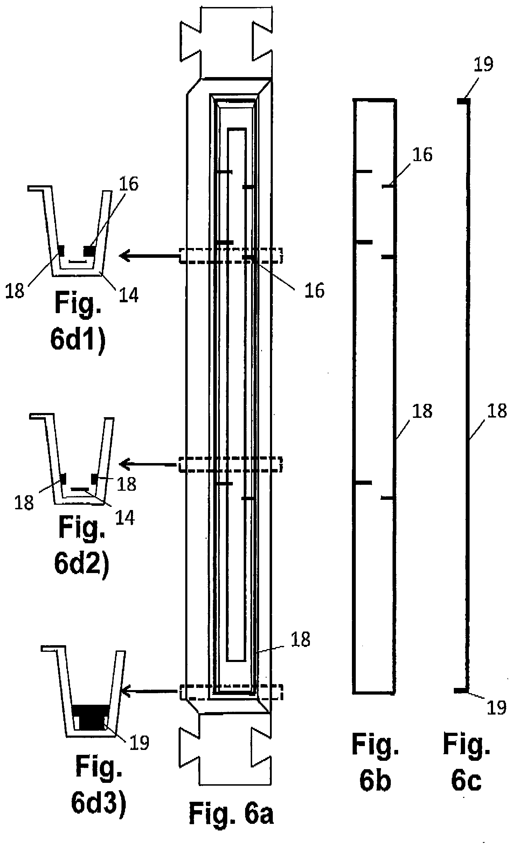

**Fig. 6** verdeutlicht die Möglichkeit, die Halteelemente in Form eines Halteelementrahmens (18) in die Inkubationsrinne einzubringen, der optional auf einem oder mehr als einem Halterahmenelementfuß (19) steht. Gezeigt sind von oben Inkubationsrinne samt Halteelementrahmen **(****Fig. 6a****)),** der Halteelementrahmen allein in gleicher Ansicht **(****Fig. 6b****)),** der Halteelementrahmen im Querschnitt entlang der Längsachse der Inkubationsrinne **(****Fig. 6c****))** sowie verschiedene Querschnitte des Halterahmens in der Inkubationsrinne, genauer an einer Stelle mit Halteelement **(****Fig. 6d1**)), ohne Halteelement **(****Fig. 6d2**)) und am Längsende durch den Halteelementfuß **(****Fig, 6d3**)).

### Bezugszeichenliste

- 1: Längliche Inkubationsrinnen
- 2 und 3: Längswände
- 4: Vertiefung
- 5: Boden
- 6: Auflagerahmen
- 7: weitere Inkubationsrinnen
- 8: Rahmenaussparung
- 9: vorstehendes Element
- 10: aufnehmendes Element
- 11: Kopf
- 12: Verbindungsstück
- 13: Aushöhlung
- 14: Teststreifen
- 15: Reagenz
- 16: Halteelement
- 17: Verbund
- 18: Halteelementrahmen
- 19: Halteelementrahmenfuß
- 20: Vorsprung
- 21: Aussparung

## Patentansprüche

1. Längliche Inkubationsrinne mit zwei einander gegenüber liegenden Längswänden, einer Vertiefung und einem Boden,
bestückt mit einem länglichen Teststreifen mit einer Rückseite und einer Vorderseite,
wobei der Teststreifen mit seiner Rückseite dem Boden der Inkubationsrinne zugewandt ist und auf seiner Vorderseite mit wenigstens einem analytischen Reagenz beschichtet ist,
weiter aufweisend ein Befestigungsmittel, über das die Inkubationsrinne mit wenigstens einer weiteren Inkubationsrinne zu einem Verbund von Inkubationsrinnen verbunden werden kann,
wobei auf einer der Längswände ein, bevorzugt waagerecht, nach außen stehender Auflagerahmen angebracht ist.

2. Inkubationsrinne nach Anspruch 1, wobei der Auflagerahmen die Vertiefung nicht verdeckt.

3. Inkubationsrinne nach einem der Ansprüche 1 bis 2, wobei die Längswand mit dem nach außen stehenden Auflagerahmen, der Auflagerahmen selbst und die andere Längswand derart aneinander angepasst sind, dass der Auflagerahmen der Inkubationsrinne im Verbund auf der anderen Längswand der weiteren Inkubationsrinne aufliegt.

4. Inkubationsrinne nach einem der Ansprüche 1 bis 3, wobei sich der Auflagerahmen entlang der Längsachse der Inkubationsrinne wenigstens so weit erstreckt wie die Vertiefung.

5. Inkubationsrinne nach einem der Ansprüche 3 bis 4, wobei sich auf der anderen Längswand eine Rahmenaussparung, bevorzugt in der Form des im Verbund darauf aufliegenden Auflagerahmens befindet.

6. Inkubationsrinne nach einem der Ansprüche 1 bis 5, wobei das Ende des Auflagerahmens entlang der Querachse so bemessen ist, dass er an die Längswand der weiteren Inkubationswanne anschließt und deren vertikale Verlängerung bildet.

7. Inkubationsrinne nach einem der Ansprüche 1 bis 6, wobei das Befestigungsmittel wenigstens eine Einheit mit einem vorstehenden Element auf der einen Längsseite der Inkubationsrinne und einem aufnehmenden Element auf der anderen Längsseite der Inkubationsrinne umfasst.

8. Inkubationsrinne nach Anspruch 7, wobei sich das vorstehende Element des Befestigungsmittels auf der gleichen Längsseite der Inkubationsrinne befindet wie der Auflagerahmen.

9. Inkubationsrinne nach einem der Ansprüche 1 bis 8, wobei das Befestigungsmittel eine Verbindung zwischen der Inkubationsrinne und der weiteren Inkubationsrinne gestattet, die *mehrfach* eingerichtet und wieder gelöst werden kann.

10. Inkubationsrinne nach einem der Ansprüche 1 bis 9, wobei an jedem Ende der Inkubationsrinne ein Befestigungsmittel, bevorzugt ein vorstehendes Element auf der einen Längsseite und ein an letzteres angepasstes aufnehmendes Element auf der anderen Längsseite, angeordnet ist.

11. Inkubationsrinne nach einem der Ansprüche 1 bis 10, wobei sich die Vertiefung nicht über die gesamte Länge der Inkubationsrinne erstreckt, sondern sich an beiden Längsenden der Inkubationsrinne ein Fortsatz befindet, der jeweils eine Einheit des Befestigungsmittel beherbergt.

12. Inkubationsrinne nach Anspruch 11, wobei jeder Fortsatz einen Kopf aufweist, der über ein Verbindungsstück mit der Vertiefung verbunden ist,
wobei das Verbindungsstück vertikal in der Ebene des Auflagerahmens angeordnet und sich unter ihm eine zur Längsachse der Inkubationsrinne senkrecht angeordnete Aushöhlung entsteht.

13. Inkubationsrinne nach einem der Ansprüche 7 bis 12, wobei
das Befestigungsmittel ein vorstehendes Element auf der einen Längsseite und ein an letzteres angepasstes aufnehmendes Element auf der anderen Längsseite umfasst,
das vorstehende Element und die Längswand mit dem Auflagerahmen an der einen Längsseite der Inkubationrinne angeordnet sind und
das aufnehmende Element und die andere Längswand an der anderen Längsseite der Inkubationsrinne angeordnet sind
und sie bevorzugt derart aneinander angepasst sind, dass die Position von wenigstens zwei gleich beschaffenen Inkubationsrinnen im Verbund sowohl durch das Einrasten des vorstehenden Elements in das aufnehmende Element als auch durch das Auflegen des Auflagerahmens auf die Rahmenaussparung der anderen Längswand fixiert ist.

14. Inkubationsrinne nach einem der Ansprüche 10 bis 13, wobei
das Befestigungsmittel ein vorstehendes Element auf der einen Längsseite und ein an letzteres angepasstes aufnehmendes Element auf der anderen Längsseite umfasst,
das aufnehmende Element des Befestigungsmittels nach oben und in Richtung der Außenseite der Inkubationsrinne offen und
so gestaltet ist, dass das vorstehende Element von oben in das aufnehmende Element eingeführt wird, bis ersteres auf den Boden des letzteren aufsetzt.

15. Inkubationsrinne nach einem der Ansprüche 10 bis 14, wobei
das Befestigungsmittel ein vorstehendes Element auf der einen Längsseite und ein an letzteres angepasstes aufnehmendes Element auf der anderen Längsseite umfasst,
und wobei das vorstehende Element und das aufnehmende Element derart aneinander angepasst sind, dass beim Verbinden das erstere mit einem hörbaren Geräusch in das letztere einrastet.

## Claims

1. An elongated incubation channel with two opposite transverse walls, a recess, and a base,
equipped with an elongated test strip with a rear surface and a front surface,
wherein the test strip has its rear surface facing the base of the incubation channel and is coated on its front surface with at least one analytical reagent,
further having a fastening means, by means of which the incubation channel can be connected to at least one additional incubation channel to form an assembly of incubation channels,
wherein a preferably horizontal outward supporting frame is installed on one of the transverse walls.

2. The incubation channel according to claim 1, wherein the supporting frame does not cover the recess.

3. The incubation channel according to any of claims 1 to 2, wherein the longitudinal wall with the outward supporting frame, the supporting frame itself, and the other longitudinal wall are adapted to one another in such a manner that the supporting frame of the incubation channel in the assembly lies on the other longitudinal wall of the additional incubation channel.

4. The incubation channel according to any of claims 1 to 3, wherein the supporting frame extends along the longitudinal axis of the incubation channel at least as far as the recess.

5. The incubation channel according to any of claims 3 to 4, wherein a frame notch is located on the other longitudinal wall, preferably in the form of the supporting frame lying thereon in the assembly.

6. The incubation channel according to any of claims 1 to 5, wherein the end of the supporting frame along the transverse axis is measured in such a way that it connects to the longitudinal wall of the additional incubation tray and forms a vertical extension thereof.

7. The incubation channel according to any of claims 1 to 6, wherein, the fastening means comprises at least one unit with a protruding element on the one longitudinal side of the incubation channel and a receiving element on the other longitudinal side of the incubation channel.

8. The incubation channel according to claim 7, wherein the protruding element of the fastening means is on the same longitudinal side of the incubation channel as the supporting frame.

9. The incubation channel according to any of claims 1 to 8, wherein the fastening means allows a connection between the incubation channel and the additional incubation channel that can be connected and disconnected multiple times.

10. The incubation channel according to any of claims 1 to 9, wherein a fastening means is configured at each end of the incubation channel, preferably with a protruding element on the one longitudinal side and a receiving element adapted thereto on the other longitudinal side.

11. The incubation channel according to any of claims 1 to 10, wherein the recess does not extend over the entire length of the incubation channel, but extensions are located at the two longitudinal ends of the incubation channel, each of which houses one unit of the fastening means.

12. The incubation channel according to claim 11, wherein each extension has a head that is connected via a connecting element with the recess,
wherein the connecting element is configured vertically at the level of the supporting frame, giving rise to a hollow that is arranged below it perpendicular to the longitudinal axis of the incubation channel.

13. The incubation channel according to any of claims 7 to 12, wherein
the fastening means comprises a protruding element on the one longitudinal side and a receiving element adapted thereto on the other longitudinal side, wherein
the protruding element and the longitudinal wall with the supporting frame are configured on the one longitudinal side of the incubation channel and
the receiving element and the other longitudinal wall are configured on the other longitudinal side of the incubation channel
and they are preferably adapted to one another such that the position of at least two identical incubation channels in the assembly is fixed both by engagement of the protruding element with the receiving element and by the supporting frame lying on the frame notch of the other longitudinal wall.

14. The incubation channel according to any of claims 10 to 13, wherein
the fastening means comprises a protruding element on the one longitudinal side and a receiving element adapted thereto on the other longitudinal side, wherein
the receiving element of the fastening means is open upwards and in the direction of the outer side of the incubation channel and
is configured in such a way that the protruding element is inserted from above into the receiving element until the former rests against the base of the latter.

15. The incubation channel according to any of claims 10 to 14, wherein
the fastening means comprises a protruding element on the one longitudinal side and a receiving element adapted thereto on the other longitudinal side,
and wherein the protruding element and the receiving element are adapted to each other in such a manner that on connection, the former engages with the latter with an audible click.

## Revendications

1. Canal d'incubation allongé avec deux parois longitudinales situées l'une en face de l'autre, une cavité et un fond, équipé d'un ruban d'étalonnage allongé avec un côté arrière et un côté avant, dans lequel le ruban d'étalonnage est tourné avec son côté arrière vers le fond du canal d'incubation et est revêtu sur son côté avant d'au moins un réactif analytique,
présentant en outre un moyen de fixation, par lequel le canal d'incubation peut être assemblé à au moins un autre canal d'incubation en un combiné de canaux d'incubation,
dans lequel un cadre d'appui dressé vers l'extérieur est installé, de préférence horizontalement, sur une des parois longitudinales.

2. Canal d'incubation selon la revendication 1, dans lequel le cadre d'appui ne recouvre pas la cavité.

3. Canal d'incubation selon une des revendications 1 à 2, dans lequel la paroi longitudinale avec le cadre d'appui dressé vers l'extérieur, le cadre d'appui lui-même et l'autre paroi longitudinale sont adaptés les uns aux autres de telle manière que le cadre d'appui du canal d'incubation repose dans le combiné sur l'autre paroi longitudinale de l'autre canal d'incubation.

4. Canal d'incubation selon l'une quelconque des revendications 1 à 3, dans lequel le cadre d'appui s'étend le long de l'axe longitudinal du canal d'incubation, au moins aussi loin que la cavité.

5. Canal d'incubation selon une des revendications 3 à 4, dans lequel il se trouve sur l'autre paroi longitudinale une découpe de cadre, de préférence sous la forme du cadre d'appui reposant sur celle-ci dans le combiné.

6. Canal d'incubation selon l'une quelconque des revendications 1 à 5, dans lequel l'extrémité du cadre d'appui le long de l'axe transversal est dimensionnée de telle manière qu'il se raccorde à la paroi longitudinale de l'autre canal d'incubation et forme le prolongement vertical de celle-ci.

7. Canal d'incubation selon l'une quelconque des revendications 1 à 6, dans lequel le moyen de fixation comprend au moins une unité avec un élément saillant sur un premier côté longitudinal du canal d'incubation et un élément de réception sur l'autre côté longitudinal du canal d'incubation.

8. Canal d'incubation selon la revendication 7, dans lequel l'élément saillant du moyen de fixation se trouve sur le même côté longitudinal du canal d'incubation que le cadre d'appui.

9. Canal d'incubation selon l'une quelconque des revendications 1 à 8, dans lequel le moyen de fixation permet une liaison entre le canal d'incubation et l'autre canal d'incubation, qui peut à plusieurs reprises être réalisée et de nouveau détachée.

10. Canal d'incubation selon l'une quelconque des revendications 1 à 9, dans lequel un moyen de fixation, de préférence un élément saillant sur un premier côté longitudinal et un élément de réception adapté à celui-ci sur l'autre côté longitudinal, est disposé à chaque extrémité du canal d'incubation.

11. Canal d'incubation selon l'une quelconque des revendications 1 à 10, dans lequel la cavité ne s'étend pas sur toute la longueur du canal d'incubation, mais il se trouve aux deux extrémités longitudinales du canal d'incubation un prolongement, qui comporte respectivement une unité du moyen de fixation.

12. Canal d'incubation selon la revendication 11, dans lequel chaque prolongement présente une tête, qui est reliée à la cavité par une pièce de liaison,
dans lequel la pièce de liaison est disposée verticalement dans le plan du cadre d'appui et il se forme en dessous d'elle une cavité disposée perpendiculairement à l'axe longitudinal du canal d'incubation.

13. Canal d'incubation selon l'une quelconque des revendications 7 à 12, dans lequel
le moyen de fixation comprend un élément saillant sur un premier côté longitudinal et un élément de réception adapté à celui-ci sur l'autre côté longitudinal,
l'élément saillant et la paroi longitudinale avec le cadre d'appui sont disposés sur le premier côté longitudinal du canal d'incubation, et
l'élément de réception et l'autre paroi longitudinale sont disposés sur l'autre côté longitudinal du canal d'incubation,
et ils sont de préférence adaptés l'un à l'autre de telle manière que la position d'au moins deux canaux d'incubation de même nature soit fixée dans le combiné aussi bien par l'encliquetage de l'élément saillant dans l'élément de réception que par la pose du cadre d'appui sur la découpe de cadre de l'autre paroi longitudinale.

14. Canal d'incubation selon l'une quelconque des revendications 10 à 13, dans lequel
le moyen de fixation comprend un élément saillant sur un premier côté longitudinal et un élément de réception adapté à celui-ci sur l'autre côté longitudinal,
l'élément de réception du moyen de fixation est ouvert vers le haut et en direction du côté extérieur du canal d'incubation et
est configuré de telle manière que l'élément saillant soit introduit par le haut dans l'élément de réception, jusqu'à ce que le premier soit appliqué contre le fond du dernier.

15. Canal d'incubation selon l'une quelconque des revendications 10 à 14, dans lequel
le moyen de fixation comprend un élément saillant sur un premier côté longitudinal et un élément de réception adapté à celui-ci sur l'autre côté longitudinal,
et dans lequel l'élément saillant et l'élément de réception sont adaptés l'un à l'autre de telle manière que lors de l'assemblage le premier se clique dans le dernier avec un bruit audible.
